# EUROPEAN PATENT SPECIFICATION

(11) **EP 2 337 580 B1**
(45) Date of publication and mention of the grant of the patent: **28.03.2012**
(21) Application number: 09782562.4
(22) Date of filing: 03.09.2009
(51) Int. Cl.: A61K 9/19, A61K 31/195, A61K 38/37

(54) **STABILIZED COMPOSITIONS FOR RECOMBINANTLY PRODUCED FACTOR VIII**
STABILISIERTE ZUSAMMENSETZUNGEN FÜR REKOMBINANT HERGESTELLTEN FAKTOR VIII
COMPOSITIONS STABILISÉES POUR LE FACTEUR VIII PRODUIT DE FAÇON RECOMBINANTE

(30) Priority: 03.09.2008 US 136402 P; 03.09.2008 EP 08163554
(43) Date of publication of application: 29.06.2011
(73) Proprietor: Octapharma AG, 8853 Lachen (CH)
(72) Inventor: RIPPNER, Brita, S-16573 Hässelby (SE); ÖSTERBERG, Josefin, S-17672 Järfälla (SE); NILSSON, Ulrika, S-16843 Bromma (SE); IVARSSON, Elsa, S-16969 Solna (SE); AGERKVIST, Irène, S-18239 Danderyd (SE)
(74) Representative: von Kreisler Selting Werner
(86) International application number: PCT/EP2009/061402
(87) International publication number: WO 2010/026186

(56) References cited:
- EP-A- 0 508 194
- EP-A- 1 652 534
- WO-A-03/080108
- DE-A1- 4 001 451

## Description

The present invention relates to lyophilized formulations with capacity to protect recombinantly produced factor VIII (r-factor VIII) of high purity. The invention also relates to liquid formulations of r-factor VIII prior to lyophilization and after reconstitution of the lyophilized solid formulation to an injectable liquid.

Factor VIII is an essential blood plasma protein involved in the blood coagulation process. Deficiency of this coagulation factor results in Hemophilia A, a life-threatening disease that must be treated by factor VIII replacement therapy. Traditionally, concentrates of purified plasma-derived factor VIII (p-factor VIII) have been used for replacement therapy. More recently, recombinantly produced factor VIII (r-factor VIII) has become available which provides a supply independently of plasma donation, reducing the risk of virus-transmitted diseases.

Factor VIII is a complex molecule and a very sensitive protein associated with activity loss over time. In the blood, other blood proteins, such as human serum albumin (HSA) and von Willebrand factor (vWF) assist in preserving the coagulant activity of factor VIII. However, it is desirable to avoid the presence of proteins obtained by purification of blood plasma in pharmaceutical formulations of r-factor VIII, because of the risk of virus transmission. Thus, it is essential to provide compositions of other pharmaceutically acceptable excipients to protect r-factor VIII against physical and chemical degradation and aggregation, which cause activity loss. A commonly used technique to prevent loss of protein activity during long-term storage is to prepare dry solid pharmaceutical formulations by lyophilization (freeze-drying). The pharmaceutical excipients must also protect factor VIII during the pharmaceutical process, during long-time storage and after reconstitution of the freeze-dried formulation to a solution for administration.

The DNA sequence of factor VIII is divided into six domains; three A-, two C- and one B-domain and the protein contains interaction sites for other clotting factors, vWF, phospholipids and metal ions. The smallest active form of the factor VIII protein lacks the B domain and is composed of a light chain of 80 kDa associated with a heavy chain of 90 kDa (Wang W. *et al,* 2003). Both full-length (Kogenate®, Bayer, Helixate®, CSL Behring, Recombinate®, Baxter and Advate®, Baxter) and B-domain deleted (ReFacto®, Wyeth and Xyntha®, Wyeth) r-factor VIII drug products are on the market today.

In a pharmaceutical formulation of factor VIII all components need to be carefully selected. Each excipient provides a protective function to keep a high yield of factor VIII throughout the pharmaceutical process, long-term storage, and finally reconstitution and administration to the patient. In addition, the clinical safety of all excipients is considered.

The purpose of the lyophilization (Manning, M. C. *et al,* 1989, Tang, X*. et al,* 2004, Schwegman, J. J. *et al,* 2005) is to remove water from the formulation, since adverse physical and chemical reactions often take place in the aqueous phase.

Cryo-/lyoprotectants are required to protect the protein during the freeze-drying process and during storage, by forming an amorphous matrix surrounding the protein.

A bulking agent is included to function as a cake former to give mechanical support during freeze-drying and to increase the dry weight of the drug product. The bulking agent thereby contributes to provide a uniform quality and appearance of a lyophilized product.

A buffering agent can be added to maintain the pH to a value suitable for the protein and for therapeutic use of the product.

Because of the high potency of factor VIII, the concentration of factor VIII in therapeutic solutions is low. In addition, factor VIII easily adsorbs to surfaces, making surface adsorption a major source of activity loss during manufacturing and after reconstitution of the product. For currently marketed factor VIII products, it is usually claimed that a surface-active agent is used above its critical micelle concentration (cmc), which is the solution concentration at which the surface-active agent form micellar aggregates. The cmc values of polyoxyethylene-containing non-ionic detergents are temperature dependent in that the cmc value becomes higher at lower temperatures (Alexandridis, P*. et al,* 1994, Nilsson , M. *et al,* 2007). The cmc of Poloxamer 188 is at least 20-30 mg/ml at 37°C (Kabanov, A. V*. et al,* 1995, Alexandridis, P*. et al,* 1994, Moghimi, S. M. *et al,* 2004) and increasing to 100 mg/ml at 20°C (Nakashima, K. *et al,* 1994). Thus, according to these reports, the cmc of Poloxamer 188 is within the interval of 20-100 mg/ml at 25°C.

Metal ions have been shown to be involved in the association of the light and heavy chains of factor VIII (Wang W *et al,* 2003) and therefore calcium ions (Ca²⁺) are normally present in formulations of factor VIII to maintain the association of the complex of the 80 and 90 kDa chains.

Considerable efforts have been made to find suitable formulations of factor VIII, for example:

A publication by Österberg *et al* (1997) describes a formulation comprising sodium chloride as bulking agent, in combination with a surfactant, calcium chloride and sucrose as stabilizer and histidine as buffering agent.

US-B-7247707 (Besman et al) discloses albumin-free formulations comprising 300 to 500 mM sodium chloride, 1 to 4% of a stabilizer chosen from the group consisting of sucrose, trehalose, raffinose and arginine, 1 to 5 mM CaCl₂, and a buffering agent, preferably histidine. A surfactant is also included in the composition, at a concentration up to 0.1%.

US-A-5874408 (Nayar) describes a formulation of recombinant factor VIII, which comprises glycine, histidine, sucrose, CaCl₂ and small amounts of sodium chloride. Nayar discovered that histidine, which is included as buffering agent in all commercially available r- factor VIII preparations today, had a de-stabilizing effect in lyophilized factor VIII formulations. This effect was however overcome by the addition of salts, glycine and sucrose.

US-A-4877608 (Lee et al) describes the use of a highly purified factor VIII protein formulation in an aqueous solution consisting essentially of therapeutically active factor VIII with an activity of at least 130 IU/mg; 0.4 to 1.2 M sodium chloride, potassium chloride or mixtures thereof; 1.5 to 40 mM calcium chloride and 1 to 50 mM histidine and optionally up to 10% sugar chosen from the group consisting of mannitol, sucrose and maltose.

US-A-2005/0256038 (White et al) teaches a lyophilized factor VIII composition comprising a surfactant, calcium chloride, sucrose, sodium chloride, trisodium citrate and a buffer devoid of amino acids.

WO-A-99/10011 (Kanellos et al*)* discloses heat-treated formulations for plasma factor VIII, with high purity. The formulation comprises a stabilizing effective amount of sucrose, trehalose and at least one amino acid. The amino acid that is preferred is lysine, but others that can be used are isoleucine, leucine, lysine, methionine, phenylalanine, threonine, tryptophan, valine, alanine, arginine, histidine, proline, serine and glycine.

EP-A-1016673 (Österberg et al) teaches the use of formulations comprising a non-ionic surfactant as a stabilizer and factor VIII having a specific activity of more than 5000 IU/mg. Further it is stated that the surfactant concentration should be above the critical micelle concentration, in an amount of at least 0.01 mg/ml.

US-B-6887852 (Paik et al*)* describes a lyophilized factor VIII formulation comprising a mixture of L-arginine, L-isoleucine and L-glutamic acid as a stabilizer. The basic formulation comprises low amounts of sodium chloride, calcium chloride and histidine. No surface-active agent is added to the composition, since the formulation as disclosed show a better stability compared to formulations comprising surfactant.

US-A-5565427 (Freudenberg) teaches the use of a stable solution of factor VIII comprising a detergent and an amino acid or one of its salts. The specific activity of the protein is at least 2000 IU/mg.

US-A-5328694 (Schwinn) discloses a stable injectable solution comprising factor VIII purified from plasma and a combination of disaccharides and one or more amino acids. Preferably, the amino acid is lysine or glycine.

The present invention relates to compositions of recombinantly produced factor VIII (r-factor VIII) of high purity. The compositions are histidine-free. In order to exert maximum protecting effects, the presently invented compositions are based on purposeful selections of excipients such as a cryo-/lyoprotectant, a bulking agent and a surface-active agent. Each added excipient may not exert its protective effects at all stages, i.e. during the pharmaceutical process, long-time storage and during reconstitution and administration.

The lyophilized formulations of the present invention are not limited to have the same filled volume and reconstitution volume. To a person skilled in the art it will be evident that the formulated product also can be reconstituted in a more dilute form.

The present invention is related with a composition according to claim 1 having a capacity to protect recombinantly produced factor VIII.

The histidine-free compositions of r-factor VIII according to the present invention generally include a combination of arginine and sucrose; a bulking agent that is sodium chloride or arginine; a surface-active agent; and optionally a pH buffering agent. The term "histidine-free" when appearing in the present contexts shall not mean compositions "devoid of histidine", since minor amounts may follow the bulk drug substance from previous manufacturing steps, but rather that no histidine has been added during the pharmaceutical processing. Histidine is frequently used as buffering agent in factor VIII compositions and while some sources report a stabilizing effect on factor VIII (EP-A-1016673, Österberg et al*),* other sources report a destabilizing effect in formulations of factor VIII (US-A-5874408, Nayar). The present invention, when appropriate, embodies sodium citrate, maleic acid or Tris (tris(hydroxymethyl)aminomethane) as a pH buffering agent. The buffering agent is e.g. sodium citrate, present in an amount to maintain a pH ranging from 6.5 to 7.5. A suitable form of the sodium citrate is the dihydrate salt. Generally, the compositions according to the invention can be in lyophilized form, but are also represented by solutions such as a solution to be lyophilized and a solution reconstituted from a lyophilized composition.

The compositions may further comprise calcium chloride in amount of about 0.5 to 10 mM to improve specific stabilization of the factor VIII molecule. The compositions can further comprise other compounds like antioxidants, such as glutathione or methionine.

A bulking agent is referred to as an excipient present in the formulation to provide mechanical support to the lyophilized cake and to increase the dry weight. The bulking agent can either be in a crystalline state, as sodium chloride, or in an amorphous state, as arginine.

A pH buffering agent is referred to as a compound with a buffering capacity in the pH range between about pH 5 and 9. The buffering capacity relates to a pKa value of the buffering agent within the said pH interval.

An ionic strength provider is referred to as an ionic compound that is present in the formulation to increase the ionic strength.

A cryo- and lyoprotectant (cryo-/lyoprotectant) is a compound present in the formulation to decrease or even prevent loss of protein activity during the freezing and drying steps of a lyophilization process and during subsequent storage of the lyophilized product.

A surface-active agent shall mean a compound that adsorbs to surfaces and interfaces and thereby counteracts activity loss of factor VIII due to adsorption. This type of activity loss may occur during the entire pharmaceutical processing as well as while handling the reconstituted product prior to and during administration to a patient. Some surface-active agents form micellar aggregates in solution. The critical micelle concentration of a surface-active agent is the concentration above which micelles are formed.

A protective composition of factor VIII means a formulation composed of selected excipients, each of the excipients providing a protective function to keep a high yield of factor VIII throughout the pharmaceutical processing, long-term storage, and finally reconstitution and administration to the patient. The pharmaceutical processing refers particularly to the last stages of the manufacturing process, starting from the arrival of bulk drug substance from production until the end of lyophilization of the formulated drug product. It should be understood that the steps of the pharmaceutical processing are generally well known to a person skilled in protein formulation and include steps like formulation, sterile filtration, filling into vials and lyophilization.

Loss of active factor VIII has a broad meaning including, but not limited to, loss due to surface adsorption, aggregation, physical and/or chemical changes of the protein structure, or loss from discarding unsatisfactory appearing lyophilizates.

The r-factor VIII, in particular is a deletion derivative fully or partially lacking the B-domain, thereby providing a specific activity which can vastly exceed 5000 IU/mg prior to formulation. Examples of such deletion derivatives fully or partially lacking their B-domains are disclosed and prepared in EP-A-1136553 (Hauser et al) and EP-A-1739179 (Schröder et al) from human cell lines. It is appreciated that the presently invented compositions, as being described in the following section, are especially well suited to protect such deletion derivatives of factor VIII.

In accordance with the present invention, the cryo-/lyoprotectant a combination of arginine and sucrose. Arginine can typically be a salt or a derivative of arginine, such as arginine hydrochloride.

The bulking agent according to the present invention also has the additional function of being an ionic strength provider, which minimizes the number of components necessary for an adequate clinical product. Bulking agents according to the present invention can be sodium chloride or arginine. Arginine can be in a salt form, in particular in the hydrochloride form.

According to the present invention, the cryo-/lyoprotectant is a combination of arginine and sucrose. The bulking agent and ionic strength provider in particular is sodium chloride. If sodium chloride acts as bulking agent, the composition of the invention is in particular comprising as a cryo/lyoprotectant 15 mg/ml of sucrose and 15 mg/ml of arginine with a proviso that at least 6 mg/ml of cryo/lyoprotectant is present, and as a bulking agent of 10 mg/ml to 40 mg/ml of sodium chloride. The composition may particularly comprise about 3 mg/ml to 10 mg/ml, particularly 4.5 mg/ml to 9 mg/ml sucrose, 3 mg/ml to about 8 mg/ml, particularly 4.5 mg/ml to 6.8 mg/ml arginine and in particular 15 mg/ml to 23 mg/ml sodium chloride. Other suitable concentration ranges comprise in particular, 4.5 to 6.8 mg/ml of sucrose, 4.5 mg/ml to 6.8 mg/ml of arginine and 15 mg/ml to 23 mg/ml sodium chloride. Preferably, arginine and sucrose are present in equal amounts. The composition comprises then up to 9 mg/ml arginine and up to 9 mg/ml sucrose. Furthermore the composition may comprise calcium chloride, a surface-active agent and, optionally, sodium citrate as pH buffering agent. In another embodiment, the composition of the invention comprises sucrose in an amount of 10 mg/ml to 25 mg/ml and sodium chloride in an amount of 10 mg/ml to 40 mg/ml.

According to another embodiment, the composition is as an alternative essentially free of sodium chloride, the cryo-/lyoprotectant is sucrose and the bulking agent and ionic strength provider is arginine. In particular, the composition comprises 5 to 25 mg/ml of sucrose and 20 to 70 mg/ml of arginine. Further this composition can comprise calcium chloride, a surface-active agent and, optionally, sodium citrate as buffering agent. The term "essentially free of sodium chloride" when appearing in the present contexts should not mean compositions "devoid of any sodium chloride", but rather contains traces of NaCl e. g. < 1 %, since minor amounts of sodium chloride may follow the bulk drug substance from earlier manufacturing steps, but rather that no sodium chloride has been added during the pharmaceutical processing.

Typically, the composition is provided in lyophilized form. In still another embodiment of the invention, the composition is provided in form of a solution to be lyophilized or in the form of a reconstituted solution prepared from a lyophilized composition and diluent.

In a further embodiment, in the composition of the invention the surface-active agent is a protein, in particular a recombinant protein. The protein is in particular recombinantly produced albumin, e.g. in an amount of 0.5 mg/ml to 5 mg/ml. This amount is considerably less than and differs from the amount commonly used in traditional formulations of plasma derived factor VIII, where the albumin functions as the only cryo/lyo protectant. Surprisingly albumin in particular recombinant albumin is very suitable for use as surface active agent in formulations of recombinant factor VIII to be stored at room temperature.

In another embodiment of the invention, the surface-active agent is a non-ionic surfactant, e.g. a polyoxyethylene-polyoxypropylene copolymer. According to the invention, the surface-active agent is present in a concentration below the critical micelle concentration, e.g. for polyoxyethylene-polyoxypropylene copolymer less than about 5 mg/ml.

In an embodiment of the invention, the composition comprises a r-factor VIII having a specific activity ≥ 5000 IU/mg protein.

According to still a further embodiment, the composition has a cryo-/lyoprotectant and a bulking agent that is arginine, which also acts as an ionic strength provider. In particular, arginine is present in an amount of 20 mg/ml to 70 mg/ml. Further this composition can comprise calcium chloride, a surface-active agent and, optionally, sodium citrate as buffering agent.

The various embodied compositions all comprise a surface-active agent that in one aspect is a non-ionic detergent, in particular a polymeric non-ionic surfactant of block copolymer type, such as a polyoxyethylene-polyoxypropylene copolymer, e.g. Poloxamer 188, or a non-ionic surfactant of polyoxyethylene sorbitan fatty acid ester type, e.g. Polysorbate 20 or Polysorbate 80. A suitable non-ionic surfactant is Poloxamer 188, which may be used at a concentration below its critical micelle concentration (cmc), preferably in particular at concentrations below about 5 mg/ml. The cmc of Poloxamer 188 has been reported to be in the range of 20-100 mg/ml at 25°C (Kabanov, A. V*. et al,* 1995, Alexandridis, P. *et al,* 1994, Moghimi, S. M*. et al,* 2004, Nakashima, K. *et al,* 1994).

In another aspect the surface-active agent is a recombinantly produced protein other than the factor VIII protein, in particular recombinant human albumin, particularly, such compositions comprise recombinantly produced albumin in an amount of 0.5 mg/ml to 5 mg/ml. The various embodiments will be described in further detail in the following examples which. illustrate the invention but should not be considered to be a restriction or a limitation of the scope of the invention.

### Examples

The factor VIII used in the experiments is a recombinant human B-domain deleted factor VIII protein, produced in the human cell line HEK293F according to the process described in EP 1739179 (Schröder et al). The purification process consisted of five chromatography steps and generated a highly pure factor VIII protein preparation (Winge *et al,* European patent application 08 158 893.1) with a human glycosylation like pattern (Sandberg *et al,* European patent application 08 162 765.5).

The protein activity was measured with a chromogenic assay or with the one stage assay.

The chromogenic assay is a two-stage photometric method that measures the biological activity of factor VIII as a cofactor. Factor VIII activates factor X into factor Xa, which in turn is enzymatically cleaved into a product that can be quantified spectrophotometrically.

The one-stage assay is based on the ability of a factor VIII containing sample to correct the coagulation time of factor VIII deficient plasma in the presence of phospholipid, contact activator and calcium ions. The time of appearance of a fibrin clot is measured in one step.

### EXAMPLE 1

The recombinant factor VIII was prepared according to the description in the experimental section above. This experiment compares a formulation having a cryo-/lyoprotectant that is a combination of arginine and sucrose with formulations having either sucrose or arginine as cryo-/lyoprotectant. Sodium chloride functions as a bulking agent and ionic strength provider.

The formulations were investigated for factor VIII recovery in lyophilized formulations, at an initial concentration of 150 IU/ml. The compositions investigated are displayed in Table I.

**TABLE I - Composition**

| | 1A | 1B | 1C |
|---|---|---|---|
| Sucrose, mg/ml | 9 | - | 9 |
| Arginine HCl, mg/ml | 9 | 9 | - |
| Sodium chloride, mg/ml | 30 | 30 | 30 |
| Calcium chloride dihydrate, mg/ml | 0.5 | 0.5 | 0.5 |
| Poloxamer 188, mg/ml | 2 | 2 | 2 |
| Sodium citrate dihydrate, mg/ml | 2 | 2 | 2 |

1.5 ml aliquots of the solutions were lyophilized in a laboratory scale freeze-drier. The lyophilized samples were stored for up to 12 months at +5°C, +25°C and +40°C to evaluate the protein activity over time. The samples were reconstituted in 1.5 ml water for injections and analyzed with the chromogenic assay, described in the experimental section above. Results are summarized in Table II.

**TABLE II - Results**

| | | Factor VIII activity over time (months), (% of initial value) | | | | | | | |
|---|---|---|---|---|---|---|---|---|---|
| | | 0 | 0.5 | 1 | 2 | 3 | 6 | 9 | 12 |
| 1A | 5°C | 100 | n.a. | 107 | n.a. | 95 | 116 | 92 | 90 |
| | 25°C | 100 | 97 | 99 | 95 | 89 | 96 | 76 | 70 |
| | 40°C | 100 | 93 | 102 | 76 | n.a. | n.a. | n.a. | n.a. |
| 1B | 5°C | 100 | n.a. | 106 | n.a. | 81 | 111 | 97 | 92 |
| | 25°C | 100 | 98 | 99 | 96 | 84 | 81 | 62 | 45 |
| | 40°C | 100 | 85 | 79 | 59 | n.a. | n.a. | n.a. | n.a. |
| 1C | 5°C | 100 | n.a. | 101 | n.a. | 86 | 102 | 88 | 82 |
| | 25°C | 100 | 85 | 86 | 80 | 83 | 82 | 71 | 63 |
| | 40°C | 100 | 70 | 66 | 56 | n.a. | n.a. | n.a. | n.a. |

| | | | | | | | | | |
|---|---|---|---|---|---|---|---|---|---|
| n.a. not analyzed | | | | | | | | | |

The results of Example 1 show that, surprisingly, there is an additive synergistic cryo-/lyoprotectant effect between sucrose and arginine, as formulation 1A shows a better activity recovery over time compared to formulations 1B and 1C.

### EXAMPLE 2

The recombinant factor VIII was prepared according to the description in the experimental section above. This experiment investigates formulations having sucrose as cryo-/lyoprotectant and arginine as bulking agent and ionic strength provider. The formulations were investigated for factor VIII recovery in lyophilized formulations, at an initial concentration of 150 IU/ml. The compositions investigated are shown in Table III.

**TABLE III - Composition**

| | 2A | 2B | 2C |
|---|---|---|---|
| Sucrose, mg/ml | 10 | 10 | 10 |
| Arginine HCl, mg/ml | 50 | 35 | 70 |
| Calcium chloride dihydrate, mg/ml | 0.5 | 0.5 | 0.5 |
| Poloxamer 188, mg/ml | 2 | 2 | 2 |
| Sodium citrate dihydrate, mg/ml | 2 | 2 | 2 |

1.5 ml aliquots of the solutions were lyophilized in a laboratory scale freeze-drier. The lyophilized samples were stored for up to 12 months at +5°C, +25°C and +40°C to evaluate the protein activity over time. The samples were reconstituted in 1.5 ml water for injections and analyzed with the chromogenic assay, as described in the experimental section above. The results are summarized in Table IV, as percentage of the initial value.

**TABLE IV - Results**

| | | Factor VIII activity over time (months), (% of initial value) | | | | | |
|---|---|---|---|---|---|---|---|
| | | 0 | 1 | 3 | 6 | 9 | 12 |
| 2A | 5°C | 100 | 94 | 80 | 92 | 89 | 101 |
| | 25°C | 100 | 94 | 84 | 86 | 74 | 84 |
| | 40°C | 100 | 90 | 81 | n.a. | n.a. | n.a. |
| 2B | 5°C | 100 | 99 | 89 | 90 | 85 | 105 |
| | 25°C | 100 | 99 | 93 | 86 | 80 | 86 |
| | 40°C | 100 | 97 | 82 | n.a. | n.a. | n.a. |
| 2C | 5°C | 100 | 97 | 88 | 93 | 88 | 97 |
| | 25°C | 100 | 93 | 78 | 87 | 76 | 80 |
| | 40°C | 100 | 90 | n.a. | n.a. | n.a. | n.a. |

| | | | | | | | |
|---|---|---|---|---|---|---|---|
| n.a. not analyzed | | | | | | | |

The results of Example 2 show that arginine functions satisfactorily as a bulking agent and ionic strength provider in combination with sucrose as a cryo-/lyoprotectant.

### EXAMPLE 3

The recombinant factor VIII was prepared according to the description in the experimental section above. This experiment compares formulations with either Poloxamer 188 or Polysorbate 80 as a surface-active agent with a formulation devoid of surface-active agent. The cryo-/lyoprotectant is a combination of arginine and sucrose, and sodium chloride is used as a bulking agent and ionic strength provider. The formulations displayed in Table V were investigated for factor VIII recovery over the lyophilization step, at an initial factor VIII concentration of 150 IU/ml.

**TABLE V - Composition**

| | 3A | 3B | 3C |
|---|---|---|---|
| Sucrose, mg/ml | 9 | 9 | 9 |
| Arginine HCl, mg/ml | 9 | 9 | 9 |
| Sodium chloride, mg/ml | 30 | 30 | 30 |
| Calcium chloride dihydrate, mg/ml | 0.5 | 0.5 | 0.5 |
| Poloxamer 188, mg/ml | 2 | - | - |
| Polysorbate 80, mg/ml | - | 0.2 | - |
| Sodium citrate dihydrate, mg/ml | 2 | 2 | 2 |

1.5 ml aliquots of the solutions were lyophilized in a laboratory scale freeze-drier. Samples were taken prior to lyophilization and frozen. Lyophilized samples were reconstituted in 1.5 ml water for injections prior to analysis. The factor VIII activity was analyzed with the chromogenic assay, described in the experimental section above. The results of the activity recovery in freeze-thawed samples and over the lyophilization step are shown in Table VI.

**TABLE VI - Results, activity recovery over the lyophilization step.**

| | Factor VIII activity, (% of added amount) | | |
|---|---|---|---|
| | Added amount | Freeze-thawed | Reconstituted after lyophilization |
| 3A | 100 | 104 | 93 |
| 3B | 100 | 101 | 97 |
| 3C | 100 | 18 | 0 |

The results of Example 3 show that a surface-active agent is needed in the formulation to counteract protein losses probably caused by surface adsorption both over a freeze-thawed step and over the lyophilization process. This example further shows that the non-ionic polymeric surface-active agent Poloxamer 188, when used at a concentration below the critical micelle concentration (cmc), effectively protects factor VIII during lyophilization. The protective effect is equally high as that of the non-ionic surface-active agent Polysorbate 80, used above its cmc value.

### EXAMPLE 4

Example 3 showed that a surface-active agent is needed in the formulation to avoid factor VIII activity loss caused by surface adsorption and this example investigates if recombinant albumin can be used for this purpose.

The recombinant factor VIII was prepared according to the description in the experimental section above. The formulations displayed in Table VII were investigated for factor VIII activity recovery in solution, at an initial factor VIII concentration of 140 IU/ml. The protein formulations were stored at +25°C and were analyzed on day 0, 3, 7 and 10 with the chromogenic assay, described in the experimental section above. The results are displayed in Table VIII, as percentage of initial value.

**TABLE VII - Composition**

| | 4A | 4B | 4C | 4D |
|---|---|---|---|---|
| Sucrose, mg/ml | 9 | 9 | 9 | 9 |
| Arginine HCl, mg/ml | 9 | 9 | 9 | 9 |
| Sodium chloride, mg/ml | 30 | 30 | 30 | 30 |
| Calcium chloride, mg/ml | 0.5 | 0.5 | 0.5 | 0.5 |
| Poloxamer 188, mg/ml | 2 | - | - | - |
| Recombinant albumin, mg/ml - | - | 1 | 2 | 4 |
| Sodium citrate, mg/ml | 2 | 2 | 2 | 2 |

**TABLE VIII - Results**

| | Factor VIII activity over time (days), (% of initial value) | | | |
|---|---|---|---|---|
| | 0 | 3 | 7 | 10 |
| 4A | 100 | 93 | 87 | 83 |
| 4B | 100 | 103 | 99 | 96 |
| 4C | 100 | 110 | 104 | 100 |
| 4D | 100 | 96 | 95 | 85 |

The results of Example 4 show that recombinant albumin can protect r-factor VIII against activity losses probably caused by surface adsorption.

### EXAMPLE 5

Example 3 showed that a surface-active agent is needed in the formulation to avoid factor VIII activity loss probably caused by surface adsorption and Example 4 showed that recombinant albumin could be used to prevent loss of protein activity in solution. This example investigates if recombinant albumin protects protein activity loss probably due to surface adsorption also in the lyophilization step.

The recombinant factor VIII was prepared according to the description in the experimental section above. The formulations are devoid of non-ionic detergent, but with recombinant albumin added to prevent activity losses. The cryo-/lyoprotectant is a combination of arginine and sucrose, and sodium chloride is used as a bulking agent and ionic strength provider. The formulations displayed in Table VII were investigated for factor VIII recovery in lyophilized formulations, at an initial factor VIII concentration of 150 IU/ml.

1.5 ml aliquots of the solutions were lyophilized in a laboratory scale freeze-drier. The lyophilized samples are stored for up to 12 months at +5°C, +25°C and +40°C to evaluate the protein activity over time. The samples are reconstituted in 1.5 ml water for injections and analyzed with the chromogenic assay, described in the experimental section above. Results are summarized in Table IX.

**TABLE IX - Results.**

| | | Factor VIII activity over time (months), (% of initial value) | | | | | |
|---|---|---|---|---|---|---|---|
| | | 0 | 1 | 3 | 6 | 9 | 12 |
| 4A | 5°C | 100 | 110 | 117 | 93 | 112 | 100 |
| | 25°C | 100 | 104 | 95 | 75 | 74 | 59 |
| | 40°C | 100 | 84 | 47 | n.a. | n.a. | n.a. |
| 4B | 5°C | 100 | 99 | 100 | 94 | 97 | 95 |
| | 25°C | 100 | 100 | 98 | 92 | 95 | 92 |
| | 40°C | 100 | 81 | 74 | n.a. | n.a. | n.a. |
| 4C | 5°C | 100 | 101 | 114 | 103 | 106 | 112 |
| | 25°C | 100 | 106 | 105 | 102 | 102 | 102 |
| | 40°C | 100 | 108 | 101 | n.a. | n.a. | n.a. |
| 4D | 5°C | 100 | 100 | 103 | 99 | 96 | 101 |
| | 25°C | 100 | 99 | 112 | 103 | 99 | 99 |
| | 40°C | 100 | 101 | 93 | n.a. | n.a. | n.a. |

| | | | | | | | |
|---|---|---|---|---|---|---|---|
| n.a. not analyzed | | | | | | | |

The results of Example 5 show that recombinant albumin can replace a non-ionic detergent in r-factor VIII formulations (formulations 4B to 4D) to avoid activity losses probably caused by surface adsorption in the lyophilization step. It also shows that recombinant albumin is very suitable for use as a surface active agent in formulations of recombinant factor VIII to be stored at room temperature.

### EXAMPLE 6

The recombinant factor VIII was prepared according to the description in the experimental section above. The formulations are devoid of non-ionic detergent, but with recombinant albumin added to prevent activity losses may be due to surface adsorption. The cryo-/lyoprotectant is sucrose, and sodium chloride is used as a bulking agent and ionic strength provider. The formulations displayed in Table X were investigated for factor VIII recovery in lyophilized formulations, at an initial factor VIII concentration of 160 IU/ml.

**TABLE X - Composition**

| | 6A | 6B |
|---|---|---|
| Sucrose, mg/ml | 24 | 24 |
| Sodium chloride, mg/ml | 30 | 30 |
| Calcium chloride, mg/ml | 0.5 | 0.5 |
| Recombinant albumin, mg/ml | 2 | 4 |
| Sodium citrate, mg/ml | 2 | 2 |

1.5 ml aliquots of the solutions were lyophilized in a laboratory scale freeze-drier. The lyophilized samples are stored for up to 6 months at +5°C, +25°C and +40°C to evaluate the protein activity over time. The samples are reconstituted in 1.5 ml water for injections and analyzed with the chromogenic assay, described in the experimental section above. Results are shown in Table XI.

**TABLE XI - Results**

| | | Factor VIII activity over time (months), (% of initial value) | | | | |
|---|---|---|---|---|---|---|
| | | 0 | 1 | 2 | 3 | 6 |
| 6A | 5°C | 100 | n.a. | n.a. | 93 | 101 |
| | 25°C | 100 | 96 | n.a. | 106 | 101 |
| | 40°C | 100 | 99 | 89 | 89 | n.a. |
| 6B | 5°C | 100 | n.a. | n.a. | 95 | 107 |
| | 25°C | 100 | 102 | n.a. | 79 | 104 |
| | 40°C | 100 | 101 | 99 | 102 | n.a. |

| | | | | | | |
|---|---|---|---|---|---|---|
| n.a. not analyzed | | | | | | |

The results of Example 6 show that recombinant albumin can replace a non-ionic detergent in r-factor VIII formulations to avoid activity losses may be caused by surface adsorption in the lyophilization step. It also shows that recombinant albumin is very suitable for use as a surface active agent in formulations of recombinant factor VIII to be stored at room temperature.

### EXAMPLE 7

This example investigates the factor VIII activity recovery in a solution containing histidine as a pH buffering agent compared with a solution devoid of pH buffering agent.

The recombinant factor VIII was prepared according to the description in the experimental section above. The solutions displayed in Table XII were investigated for factor VIII activity recovery in solution, at an initial factor VIII concentration of 100 IU/ml.

**TABLE XII- Composition**

| | 7A | 7B |
|---|---|---|
| Sodium chloride, mg/ml | 18 | 18 |
| Calcium chloride dihydrate, mg/ml | 0.5 | 0.5 |
| Polysorbate 80, mg/ml | 0.2 | 0.2 |
| Histidine, mg/ml | 3 | - |

The protein formulations were stored at +25°C and were analyzed on day 0 and after 3 and 7 days with the chromogenic assay, described in the experimental section above. The results are displayed in Table XIV, as percentage of initial value.

**TABLE XIV - Results**

| | | Factor VIII activity over time (days), (% of initial value) | | |
|---|---|---|---|---|
| | | 0 | 3 | 7 |
| 7A | 25°C | 100 | 81 | 72 |
| 7B | 25°C | 100 | 95 | 90 |

The results of Example 7 show that a formulation free of histidine protects the factor VIII better than a formulation containing histidine.

### EXAMPLE 8

The recombinant factor VIII was prepared according to the description in the experimental section above. This experiment investigates formulations having arginine as both cryo-/lyoprotectant, bulking agent and ionic strength provider. The formulations were investigated for factor VIII recovery in lyophilized formulations, at an initial concentration of 160 IU/ml. The compositions investigated are shown in Table XV.

**TABLE XV - Composition**

| | 8A |
|---|---|
| Arginine HCl, mg/ml | 70 |
| Calcium chloride dihydrate, mg/ml | 0.5 |
| Poloxamer 188, mg/ml | 2 |
| Sodium citrate dihydrate, mg/ml | 2 |

1.5 ml aliquots of the solutions were lyophilized in a laboratory scale freeze-drier. The lyophilized samples were stored for up to 9 months at +5°C, +25°C and +40°C to evaluate the protein activity over time. The samples were reconstituted in 1.5 ml water for injections and analyzed with the chromogenic assay, as described in the experimental section above. The results are summarized in Table XVI, as percentage of the initial value.

**TABLE XVI - Results**

| | | Factor VIII activity over time (months), (% of initial value) | | | | | |
|---|---|---|---|---|---|---|---|
| | | 0 | 1 | 2 | 3 | 6 | 9 |
| 8A | 5°C | 100 | 88 | n.a. | 98 | 85 | 92 |
| | 25°C | 100 | 83 | n.a. | 97 | 81 | 80 |
| | 40°C | 100 | 85 | 79 | 85 | n.a. | n.a. |

| | | | | | | | |
|---|---|---|---|---|---|---|---|
| n.a. not analyzed | | | | | | | |

The results of Example 8 show that arginine can be a multifunctional excipient since it functions satisfactorily as both bulking agent and ionic strength provider, as well as cryo/lyoprotectant.

### Reference list

Wang, W., Wang, Y. W. and Kelner, D. N., Coagulation factor VIII: structure and stability (Review), Int. J. Pharm., 259, (2003), 1-15
Schwegman, J. J., Hardwick, L. M. and Akers, M. J., Practical Formulation and process Development of Freeze-Dried Products, Pharm. Dev. and Techn., 10, (2005), 151-173
Tang, X. and Pikal, M. J., Design of Freeze-Drying Processes for Pharmaceuticals: Practical Advice, Pharm. Res., 21 (2), (2004), 191-200
Manning, M. C., Patel, K. and Borchardt, R. T., Stability of Protein Pharmaceuticals, Pharm. Res., 6 (11), (1989), 903-918
Österberg, T., Fatouros, A. and Mikaelsson, M., Development of a Freeze-Dried Albumin-Free Formulation of Recombinant Factor VIII SQ, Pharm. Res., 14, (1997), 892-898
Alexandridis, P. et al, Micellization of Poly(ethylene oxide)-Poly(propylene oxide)-Poly(ethylene oxide) Triblock Copolymers in Aqueous Solutions: Thermodynamics of Copolymer Association, Macromolecules, 27, (1994), 2414-2425
Kabanov, A. V. et al, Micelle Formation and Solubilization of Fluorescent Probes in Poly(oxyethylene-b-oxypropylene-b-oxyethylene) Solutions,
Macromolecules, 28, (1995), 2303-2314
Moghimi, S. M. et al, Biochimica et Biophysica Acta, 2004, 1689, 103-113
Nakashima, K. et al, Fluorescence Studies on the Properties of a Pluronic F68
Micelle, Langmuir, 10, (1994), 658-661
Nilsson, M. et al, Influence of Polydispersity on the Micellization of Triblock Copolymers Investigated by Pulsed Field Gradient Nuclear Magnetic Resonance, Macromolecules, 40, (2007), 8250-8258

## Claims

1. A histidine-free composition comprising:
a high purity factor VIII or r-factor VIII;
arginine and sucrose;
a surface-active agent to prevent or at least inhibit surface adsorption of factor VIII;
an amount of calcium chloride for specific stabilization of factor VIII.

2. The histidine-free composition of claim 1 wherein sodium chloride is present as bulking agent if arginine and sucrose are working as cryo/lyoprotectant but not as bulking agent.

3. The histidine-free composition of claim 1 wherein essentially no sodium chloride is present, in particular if arginine is used as cryo/lyoprotectant and bulking agent.

4. The composition according to any one of claims 1 to 3 wherein the r-factor VIII is a deletion derivative of native factor VIII, partially, or entirely lacking the B-domain of native factor VIII.

5. The composition according to any one of claims 1 to 4 in lyophilized form.

6. The composition according to any one of claims 1 to 4 in form of a solution to be lyophilized or in the form of a reconstituted solution prepared from a lyophilized composition and diluent.

7. The composition according to any one of claims 2, and 4 to 6 comprising as a cryo/lyoprotectant 3-15 mg/ml of sucrose and 3-15 mg/ml of arginine with a proviso that at least 6 mg/ml of cryo/lyoprotectant are present, and as a bulking agent 10 mg/ml to 40 mg/ml of sodium chloride.

8. The composition according to claim 7 wherein the cryo/lyoprotectant includes both arginine and sucrose, in particular 3 mg/ml to 10 mg/ml, particularly 4.5 mg/ml to 9 mg/ml sucrose, 3 mg/ml to 8 mg/ml, particularly 4.5 mg/ml to 6.8 mg/ml arginine and 10 to 40 mg/ml NaCl, in particular 15 mg/ml to 23 mg/ml sodium chloride.

9. The composition of claim 1 or 2 wherein sucrose is present in an amount of 10 mg/ml to 25 mg/ml and sodium chloride is present of an amount of 10 mg/ml to 40 mg/ml.

10. The composition according to any one of claims 3 to 6 comprising a cryo/lyoprotectant that is sucrose and a bulking agent that is arginine, in particular 5 mg/ml to 25 mg/ml of sucrose and 20 mg/ml to 70 mg/ml of arginine.

11. The composition according to any one of claims 3 to 6 wherein arginine functions as both bulking agent and cryo/lyoprotectant, in particular in an amount of 20 mg/ml to 70 mg/ml.

12. The composition according to any one of claims 1 to 11 wherein the surface-active agent is a protein, in particular a recombinant protein such as recombinantly produced albumin, in particular in an amount of 0.5 mg/ml to 5 mg/ml.

13. The composition according to any one of claims 1 to 11 wherein the surface-active agent is a non-ionic surfactant, in particular in a concentration below the critical micelle concentration.

14. The composition according to claim 13 wherein the non-ionic surfactant is a polyoxyethylene-polyoxypropylene copolymer, in particular in a concentration of 0.1 mg/ml to 5 mg/ml.

15. The composition according to any one of claim 1 to 14 wherein the specific activity of r-factor VIII is ≥ 5000 IU/mg protein.

## Patentansprüche

1. Histidinfreie Zusammensetzung, umfassend:
einen hochreinen Faktor VIII oder rekombinanten Faktor VIII;
Arginin und Saccharose;
ein Tensid, um die Oberflächenadsorption von Faktor VIII zu verhindern oder wenigstens zu hemmen;
eine Menge an Calciumchlorid für die spezifische Stabilisierung von Faktor VIII.

2. Histidinfreie Zusammensetzung gemäß Anspruch 1, wobei Natriumchlorid als Füllstoff vorhanden ist, wenn Arginin und Saccharose als Kryo-/Lyoprotektor, aber nicht als Füllstoff dienen.

3. Histidinfreie Zusammensetzung gemäß Anspruch 1, wobei im Wesentlichen kein Natriumchlorid vorhanden ist, insbesondere wenn Arginin als Kryo-/Lyoprotektor und Füllstoff verwendet wird.

4. Zusammensetzung gemäß einem der Ansprüche 1 bis 3, wobei der rekombinante Faktor VIII ein Deletionsderivat von nativem Faktor VIII ist, dem teilweise oder ganz die B-Domäne des nativen Faktor VIII fehlt.

5. Zusammensetzung gemäß einem der Ansprüche 1 bis 4 in lyophilisierter Form.

6. Zusammensetzung gemäß einem der Ansprüche 1 bis 4 in Form einer zu lyophilisierenden Lösung oder in Form einer rekonstituierten Lösung, die aus einer lyophilisierten Zusammensetzung und Verdünnungsmittel hergestellt wurde.

7. Zusammensetzung gemäß einem der Ansprüche 2 und 4 bis 6, die als Kryo-/Lyoprotektor 3-15 mg/ml Saccharose und 3-15 mg/ml Arginin, mit der Maßgabe, dass wenigstens 6 mg/ml Kryo-/Lyoprotektor vorhanden sind, und als Füllstoff 10 mg/ml bis 40 mg/ml Natriumchlorid umfasst.

8. Zusammensetzung gemäß Anspruch 7, wobei der Kryo-/Lyoprotektor sowohl Arginin als auch Saccharose, insbesondere 3 mg/ml bis 10 mg/ml, insbesondere 4,5 mg/ml bis 9 mg/ml, Saccharose, 3 mg/ml bis 8 mg/ml, insbesondere 4,5 mg/ml bis 6,8 mg/ml, Arginin, und 10 bis 40 mg/ml NaCl, insbesondere 15 mg/ml bis 23 mg/ml Natriumchlorid, umfasst.

9. Zusammensetzung gemäß Anspruch 1 oder 2, wobei Saccharose in einer Menge von 10 mg/ml bis 25 mg/ml vorhanden ist und Natriumchlorid in einer Menge von 10 mg/ml bis 40 mg/ml vorhanden ist.

10. Zusammensetzung gemäß einem der Ansprüche 3 bis 6, umfassend einen Kryo-/Lyoprotektor, bei dem es sich um Saccharose handelt, und einen Füllstoff, bei dem es sich um Arginin handelt, insbesondere 5 mg/ml bis 25 mg/ml Saccharose und 20 mg/ml bis 70 mg/ml Arginin.

11. Zusammensetzung gemäß einem der Ansprüche 3 bis 6, wobei Arginin sowohl als Füllstoff als auch als Kryo-/Lyoprotektor dient, insbesondere in einer Menge von 20 mg/ml bis 70 mg/ml.

12. Zusammensetzung gemäß einem der Ansprüche 1 bis 11, wobei das Tensid ein Protein ist, insbesondere ein rekombinantes Protein, wie rekombinant hergestelltes Albumin, insbesondere in einer Menge von 0,5 mg/ml bis 5 mg/ml.

13. Zusammensetzung gemäß einem der Ansprüche 1 bis 11, wobei das Tensid ein nichtionisches Tensid ist, insbesondere in einer Konzentration unterhalb der kritischen Mizellbildungskonzentration.

14. Zusammensetzung gemäß Anspruch 13, wobei das nichtionische Tensid ein Polyoxyethylen-Polyoxypropylen-Copolymer ist, insbesondere in einer Konzentration von 0,1 mg/ml bis 5 mg/ml.

15. Zusammensetzung gemäß einem der Ansprüche 1 bis 14, wobei die spezifische Aktivität des rekombinanten Faktors VIII ≥ 5000 IE/mg Protein ist.

## Revendications

1. Composition dépourvue d'histidine comprenant :
un facteur VIII ou facteur r VIII de haute pureté ;
de l'arginine et du saccharose ;
un agent tensioactif pour prévenir ou au moins inhiber l'adsorption de surface du facteur VIII ;
une quantité de chlorure de calcium pour stabilisation spécifique du facteur VIII.

2. Composition dépourvue d'histidine selon la revendication 1, dans laquelle du chlorure de sodium est présent en tant que diluant si l'arginine et le saccharose fonctionnent comme un cryo/lyoprotecteur mais pas comme un agent d'étoffement.

3. Composition dépourvue d'histidine selon la revendication 1, dans laquelle essentiellement aucun chlorure de sodium n'est présent, en particulier si de l'arginine est utilisée comme cryo/lyoprotecteur et agent d'étoffement.

4. Composition selon l'une quelconque des revendications 1 à 3, dans laquelle le facteur r VIII est un dérivé de délétion du facteur VIII natif manquant partiellement ou entièrement du domaine B du facteur VIII natif.

5. Composition selon l'une quelconque des revendications 1 à 4, sous forme lyophilisée.

6. Composition selon l'une quelconque des revendications 1 à 4, sous forme d'une solution à lyophiliser ou sous la forme d'une solution reconstituée préparée à partir d'une composition lyophilisée et d'un diluant.

7. Composition selon l'une quelconque des revendications 2, et 4 à 6, comprenant comme cryo/lyoprotecteur, 3 à 15 mg/mL de saccharose et 3 à 15 mg/mL d'arginine à condition qu'au moins 6 mg/mL de cryo/lyoprotecteur soient présents, et comme agent d'étoffement, 10 mg/mL à 40 mg/mL de chlorure de sodium.

8. Composition selon la revendication 7, dans laquelle le cryo/lyoprotecteur inclut à la fois de l'arginine et du saccharose, en particulier 3 mg/mL à 10 mg/mL, en particulier 4,5 mg/mL à 9 mg/mL de saccharose, 3 mg/mL à 8 mg/mL, en particulier 4,5 mg/mL à 6,8 mg/mL d'arginine et 10 à 40 mg/mL de NaCl, en particulier 15 mg/mL à 23 mg/mL de chlorure de sodium.

9. Composition selon la revendication 1 ou 2, dans laquelle du saccharose est présent en une quantité de 10 mg/mL à 25 mg/mL et du chlorure de sodium est présent en une quantité de 10 mg/mL à 40 mg/mL.

10. Composition selon l'une quelconque des revendications 3 à 6, comprenant un cryo/lyoprotecteur qui est le saccharose et un agent d'étoffement qui est l'arginine, en particulier 5 mg/mL à 25 mg/mL de saccharose et 20 mg/mL à 70 mg/mL d'arginine.

11. Composition selon l'une quelconque des revendications 3 à 6, dans laquelle l'arginine fonctionne à la fois comme agent d'étoffement et cryo/lyoprotecteur, en particulier dans une quantité de 20 mg/mL à 70 mg/mL.

12. Composition selon l'une quelconque des revendications 1 à 11, dans laquelle l'agent tensioactif est une protéine, en particulier une protéine recombinante telle que de l'albumine produite de façon recombinante, en particulier dans une quantité de 0,5 mg/mL à 5 mg/mL.

13. Composition selon l'une quelconque des revendications 1 à 11, dans laquelle l'agent tensioactif est un tensio-actif non ionique, en particulier à une concentration en dessous de la concentration micellaire critique.

14. Composition selon la revendication 13, dans laquelle le tensio-actif non ionique est un copolymère de poly(oxyéthylène)-poly(oxypropylène), en particulier à une concentration de 0,1 mg/mL à 5 mg/mL.

15. Composition selon l'une quelconque des revendications 1 à 14, dans laquelle l'activité spécifique du facteur r VIII est ≥ 5 000 UI/mg de protéine.
